# EUROPÄISCHE PATENTSCHRIFT

(11) **EP 2 780 896 B1**
(45) Veröffentlichungstag und Bekanntmachung des Hinweises auf die Patenterteilung: **10.01.2018**
(21) Anmeldenummer: 12798626.3
(22) Anmeldetag: 14.11.2012
(51) Int. Cl.: G07C 9/00

(54) **INTEGRIERTER FINGERABDRUCK- UND GEFAHRSTOFF-SENSOR, AUTOMATISCHE PERSONENSCHLEUSE MIT EINEM SOLCHEN INTEGRIERTEN SENSOR SOWIE VERWENDUNG EINES SOLCHEN SENSORS ZUR SICHERHEITSKONTROLLE IN EINEM FLUGHAFEN**
INTEGRATED FINGERPRINT AND HAZARDOUS MATERIAL SENSOR, AUTOMATIC ENTRY CONTROL FOR PEOPLE, SAID ENTRY CONTROL COMPRISING SUCH AN INTEGRATED SENSOR, AND USE OF SUCH A SENSOR FOR SECURITY CHECKPOINTS IN AN AIRPORT
DÉTECTEUR INTÉGRÉ D'EMPREINTES DIGITALES ET DE MATIÈRES DANGEREUSES, BARRIÈRE DE SÉCURITÉ AUTOMATIQUE PRÉSENTANT UN TEL DÉTECTEUR INTÉGRÉ, ET UTILISATION D'UN TEL DÉTECTEUR POUR LE CONTRÔLE DE SÉCURITÉ DANS UN AÉROPORT

(30) Priorität: 16.11.2011 DE 102011118666
(43) Veröffentlichungstag der Anmeldung: 24.09.2014
(73) Patentinhaber: AIRSENSE Analytics GmbH, 19061 Schwerin (DE)
(72) Erfinder: GOTTHARDT, Cordula, 8309 Nürensdorf (CH); LÖBAU, Jörg, 88633 Heiligenberg (DE); WALTE, Andreas, 19061 Schwerin (DE)
(74) Vertreter: Maiwald Patentanwalts GmbH
(86) Internationale Anmeldenummer: PCT/EP2012/072615
(87) Internationale Veröffentlichungsnummer: WO 2013/072364

(56) Entgegenhaltungen:
- WO-A1-2006/096246
- WO-A2-2007/086913
- US-A1- 2004 227 929
- US-A1- 2006 042 407

## Beschreibung

### Gebiet der Erfindung

Die Erfindung betrifft die Gefahrstoffdetektion. Insbesondere betrifft die Erfindung einen integrierten Fingerabdruck- und Gefahrstoff-Sensor, eine automatische Personenschleuse und die Verwendung eines integrierten Fingerabdruck- und Gefahrstoff-Sensors zur Sicherheitskontrolle in einem Flughafen.

### Technischer Hintergrund

Gefahrstoffe, wie beispielsweise Explosivstoffe, Gifte, Drogen, bioaktive oder radioaktive Substanzen, können mit geeigneten Sensoren detektiert werden. Um die Identität der mit dem detektierten Gefahrstoff zusammenhängenden Person feststellen zu können, muss beispielsweise deren Reisepass kontrolliert werden.

Ein solches Kontrollverfahren kann zu Verzögerungen führen, was insbesondere bei der Abfertigung von Fluggästen oft ungewünscht ist. Die Druckschrift US 2006/0042407 offenbart einen integrierten Fingerabdruck- und Gefahrstoff-Sensor zur Sicherheitskontrolle von Personen. Der Fingerabdruck-Leser nimmt einen Fingerabdruck auf, indem ein Finger von außen über einen Schlitz einer drehbaren Trommel bewegt wird. Nach einer solchen Aufnahme wird der Schlitz durch verdrehen der Trommel einem Desorber und einer Probentransferbox zur Analyse des Schlitzbereichs auf Gefahrstoffe zugeführt.

### Zusammenfassung der Erfindung

Es kann als eine Aufgabe der Erfindung angesehen werden, die Sicherheitskontrolle von Personen effizient zu gestalten.

Gemäß einem ersten Aspekt der Erfindung ist ein integrierter Fingerabdruck- und Gefahrstoff-Sensor in Form einer Sensor- und Scanneranordnung zur Sicherheitskontrolle von Personen angegeben, der eine Fingerabdruck- und/oder Handflächen-Scaneinrichtung und eine Gefahrstoff-Sensoreinrichtung aufweist. Die Fingerabdruck- oder Handflächen-Scaneinrichtung kann einen Fingerabdruck und/oder einen Handabdruck einer Person aufnehmen und die Gefahrstoff-Sensoreinrichtung kann einen Gefahrstoff detektieren, der von der Person, also z.B. von dem Finger oder von der Hand der Person, während der Aufnahme des Abdrucks des Fingers bzw. der Hand an die Fingerabdruck- oder Handflächen-Scaneinrichtung abgegeben wurde.

Diese Stoffabgabe kann erfolgen, wenn der Finger bzw. die Hand auf die Oberfläche der Scaneinrichtung gelegt wird, so dass der sich auf der Haut der Person befindliche Gefahrstoff von der Oberfläche der Scaneinrichtung (beispielsweise einer Glasplatte) adsorbiert wird.

Ein weiterer Aspekt der Erfindung betrifft eine automatische Personenschleuse zur Sicherheitskontrolle von Personen, welche einen oben und im Folgenden beschriebenen integrierten Fingerabdruck- und Gefahrstoff-Sensor aufweist.

Ein weiterer Aspekt der Erfindung betrifft die Verwendung eines oben und im Folgenden beschriebenen Fingerabdruck- und Gefahrstoff-Sensors zur Sicherheitskontrolle in einem Flughafen und/oder zur Zugangskontrolle zu einem geschützten Bereich eines Flughafens oder einer anderen Einrichtung, beispielsweise ein Regierungsgebäude, ein Atomkraftwerk, etc. Insbesondere kann der Sensor tragbar ausgeführt sein, wodurch er sich zum mobilen Einsatz eignet. Für diesen Anwendungsfall kann er, zum Datenaustausch mit einer Datenverarbeitungsanlage, über eine Drahtlosschnittstelle verfügen.

Ein Kemaspekt der Erfindung kann darin gesehen werden, dass der integrierte Sensor mehrere Detektoren oder Sensoren aufweist, welche unterschiedliche Informationen erfassen können.

Zum einen kann so die Identität der zu untersuchenden Person festgestellt werden. Dies erfolgt durch den Fingerabdruck- und Gefahrstoff-Sensor. Zusätzlich hierzu kann gemäß einem weiteren Aspekt der Erfindung ein Dokumentenlesegerät vorgesehen sein, beispielsweise ein Pass- oder Personalausweis-Scanner, um weitere Informationen hinsichtlich der Identität der Person erfassen zu können. Die Fingerabdruck- oder Handflächen-Scaneinrichtung und der Dokumentenscanner können in Kombination miteinander arbeiten, um beispielsweise Widersprüche zwischen den verschiedenen Personeninformationen feststellen zu können. So könnte beispielsweise auch ein gefälschter oder falscher Pass als solcher identifiziert werden, wenn nämlich der Fingerabdruck nicht zur Identität der Person, die in dem Pass genannt ist, passt.

Zum anderen ist eine Gefahrstoff-Sensoreinrichtung vorgesehen, welche in der Lage ist, geringe Spuren eines von der Hand bzw. dem Finger der Person abgegebenen Gefahrstoffes zu detektieren. Der Gefahrstoff wird auf die Oberfläche der Scaneinrichtung (oder einer anderen, dafür vorgesehenen Oberfläche) abgegeben, wenn die Person diese Oberfläche berührt. Daraufhin detektiert die Gefahrstoff-Sensoreinrichtung bereits kleinste Spuren dieses Gefahrstoffes bzw. verschiedener, in der Stoffprobe enthaltener Gefahrstoffe.

Hierfür kann die Gefahrstoff-Sensoreinrichtung einen oder mehrere gleiche oder verschiedenartige Sensoren aufweisen, welche gleiche oder verschiedene Gefahrstoffe detektieren können.

Durch die integrierte Ausführung des Fingerabdruck- und Gefahrstoff-Sensors ist es möglich, die verschiedenen Messungen in einem Gerät durchzuführen, und das in kurzer Zeit, ohne dass der Benutzer hierdurch beeinträchtigt wird und teilweise auch, ohne dass dies der Benutzer feststellen kann. Der Benutzer muss lediglich seinen Finger- oder Handabdruck abgeben. Die weiteren Messungen laufen automatisch im Hintergrund.

Insbesondere können die Messungen innerhalb einer Personenschleuse vorgenommen werden, welche zur Zugangskontrolle in einen gesicherten Bereich eingesetzt wird. Hat die Person die Schleuse betreten, wird der Fingerabdruck bzw. Handabdruck abgegeben und die weiteren Messungen werden durchgeführt. Auch kann die automatische Personenschleuse eine Dokumenten-Scaneinrichtung zum Einlesen und Überprüfen von Personendaten aus einem Dokument (Pass, Personalausweis, Führerschein, etc.) aufweisen.

Insbesondere kann die Schleuse ausgeführt sein, den Durchgang der Person durch die Schleuse zu verhindern, wenn der Fingerabdruck- und Gefahrstoff-Sensor (bei dem es sich um mindestens zwei verschiedene Sensor- bzw. Scaneinrichtungen handelt) einen Gefahrstoff detektiert. In diesem Fall kann beispielsweise ein stiller oder, je nach Gefährlichkeit und Konzentration des Gefahrstoffes und/oder je nach Gefährlichkeit der identifizierten Person, akustischer oder optischer Alarm abgegeben werden, durch den das Sicherheitspersonal informiert wird. Darüber hinaus kann in diesem Fall die Schleuse verriegelt werden bzw. verriegelt bleiben, so dass die Person nicht entfliehen kann.

Gemäß einem weiteren Aspekt der Erfindung ist die automatische Personenschleuse zur Verhinderung des Durchgangs der Person durch die Schleuse lediglich dann ausgeführt, wenn die Konzentration des detektierten Gefahrstoffs eine Mindestkonzentration übersteigt. Gemäß einem weiteren Aspekt der Erfindung weist die Fingerabdruck- oder Handflächen-Scaneinrichtung eine Oberfläche zum Anlegen des Fingers oder der Hand während der Aufnahme des Abdrucks auf, auf welcher sich der Gefahrstoff bei Anlegen des Fingers oder der Hand ablagert, wobei die Gefahrstoff-Sensoreinrichtung zur anschließenden, automatischen Identifikation des Gefahrstoffs ausgeführt ist.

Somit laufen die sich an der Messung des Fingerabdrucks oder Handabdrucks anschließenden Messungen automatisch ab, ohne dass hierfür bestimmte Handlungen der zu untersuchenden Person erforderlich sind.

Gemäß einem weiteren Aspekt der Erfindung weist der integrierte Sensor eine Transportvorrichtung zum Transport der Fingerabdruck- oder Handflächen-Scaneinrichtung in einen Wirkbereich der Gefahrstoff-Sensoreinrichtung auf, nachdem die Aufnahme des Abdrucks erfolgt ist.

Auch ist vorgesehen, gemäß einem weiteren Aspekt der Erfindung, dass die Transportvorrichtung zum Transport der Gefahrstoff-Sensoreinrichtung in Richtung der Fingerabdruck- oder Handflächen-Scaneinrichtung ausgeführt ist, so dass die Fingerabdruck- oder Handflächen-Scaneinrichtung in einen Wirkbereich der Gefahrstoff-Sensoreinrichtung gelangt, nachdem die Aufnahme des Abdrucks erfolgt ist.

Auch können sich beide Vorrichtungen (Fingerabdruck- oder Handflächen-Scaneinrichtung und Gefahrstoff-Sensoreinrichtung) mittels zweier Transportvorrichtungen aufeinander zu bewegen.

Somit wird sichergestellt, dass die Sensoreinrichtung zur Analyse der von der Person abgegebenen Stoffprobe nahe genug an die Oberfläche der Scaneinrichtung herangeführt werden kann (oder umgekehrt).

Gemäß einem weiteren Aspekt der Erfindung weist die Transportvorrichtung hierfür einen Drehteller auf, auf welchem die Fingerabdruck- oder Handflächen-Scaneinrichtung angeordnet ist und mit welchem sie in den Wirkbereich der Gefahrstoff-Sensoreinrichtung hineingedreht wird.

Hierbei kann die durch die Scaneinrichtung erfasste Position der Finger auf der Oberfläche der Scaneinrichtung (also z.B. der Glasoberfläche) zum Ausrichten der Desorptions- oder Detektionsvorrichtung (Sensoreinrichtung) ausgenutzt werden, um gezielt nur die vom Finger stammenden Gefahrstoffe zu desorbieren.

Gemäß einem weiteren Aspekt der Erfindung ist also die Gefahrstoff-Sensoreinrichtung zur Durchführung eines Thermo desorptions verfahren zur Überführung des schwerflüchtigen Gefahrstoffs auf die Fingerabdruck- oder Handflächen-Scaneinrichtung in eine Gasphase zur weiteren Analyse des Gefahrstoffs ausgeführt.

Alternativ zur Thermodesorption kann die Überführung des schwerflüchtigen Gefahrstoffs in die Gasphase auch mittels einer selektiven Anregung durch Strahlung im Absorptionsbereich des Gefahrstoffs erfolgen. Auf diese Weise kann eine höhere Selektivität durch gezielte Desorption des interessierenden Gefahrstoffs erreicht werden. Alternativ kann die Desorption auch mit gleichzeitiger Ionisierung der Moleküle des Gefahrstoffs einhergehen.

Gemäß einem weiteren nicht beanspruchten Aspekt saugt die Gefahrstoff-Sensoreinrichtung gasförmige Gefahrstoffe im Bereich der Fingerabdruck- oder Handflächen-Scaneinrichtung zur weiteren Analyse des Gefahrstoffs ab.

Gemäß einem weiteren nicht beanspruchten Aspekt können gasförmige Gefahrstoffe, welche von Handflächen oder Kleidungsstücken ausgasen, zusätzlich angesaugt und zur weiteren Analyse des Gefahrstoffs überführt werden.

Gemäß einem weiteren Aspekt der Erfindung ist die Gefahrstoff-Sensoreinrichtung zur Durchführung einer chemischen und/oder elektrochemischen Analyse des Gefahrstoffs ausgeführt.

Gemäß einem weiteren Aspekt der Erfindung ist die Gefahrstoff-Sensoreinrichtung zur Detektion von radioaktiver Strahlung, die von dem Gefahrstoff emittiert wird, ausgeführt.

Gemäß einem weiteren Aspekt der Erfindung ist die Gefahrstoff-Sensoreinrichtung zur Detektion von radioaktiver Alpha- und/oder Beta-Strahlung ausgeführt, so dass auch eine Detektion von radioaktiven Gefahrstoffen ermöglicht werden kann, welche keine oder nur sehr energiearme Gamma-Strahlung aussenden. Insbesondere kann auf diese Weise z.B. Plutonium detektiert werden.

Gemäß einem weiteren Aspekt der Erfindung ist die Gefahrstoff-Sensoreinrichtung zur Detektion von bioaktiven Substanzen ausgeführt.

Darüber hinaus kann ein zusätzlicher Gamma-Detektor vorgesehen sein, der beispielsweise nicht in der Gefahrstoff-Sensoreinrichtung des integrierten Fingerabdruck- und Gefahrstoff-Sensors integriert ist, sondern sich an einem anderen Ort (beispielsweise innerhalb der automatischen Personenschleuse) befindet und zum Nachweis von nicht Alpha- und Beta-strahlenden Nukliden ausgeführt ist.

An dieser Stelle sei angemerkt, dass die Gefahrstoff-Sensoreinrichtung mehrere Sensoren aufweisen kann, welche jeweils eine der oben beschriebenen Detektionsverfahren durchführen können.

Darüber hinaus kann, gemäß einem weiteren nicht beanspruchten Aspekt, die automatische Personenschleuse einen Bordkartenleser zum Einlesen einer Bordkarte der Person aufweisen, um beispielsweise dieser Person den Zugang zur Schleuse zu ermöglichen.

Die Abnahme eines weiteren Finger- oder Handabdrucks ist nicht erforderlich. Vorteilhaft ist weiterhin, dass, da ein Finger-Scan-Bild der ganzen Handfläche vorliegen kann, gezielt die Fläche der transparenten Platte für eine Detektion genutzt werden kann, die ihrer Wahrscheinlichkeit nach am meisten Partikel aufweisen wird, also z.B. dort wo der Ringfinger gelegen hat.

Hieraus können sich die folgenden Vorteile ergeben:
- Schnellere Detektion, da nicht die ganze Fläche gescannt werden muss.
- Gezielte Probennahme, da ja bekannt ist, wo der Finger auf der Fläche gelegen hat. Auf diese Weise kann die Fehlerwahrscheinlichkeit herabgesetzt werden.
- Glas ist analytisch sehr interessant, da es keine Eigenstofflichkeit zeigt, also keine Stoffe absondert, welche die Detektion stören könnten. Außerdem ist Glas sehr gut zu reinigen und weißt gegenüber den meisten anderen Materialien keine Alterungserscheinung auf.
- Glas ist auch weitgehend hitzeresistent, was bei dessen Verwendung in einem Thermodesorptionsverfahren günstig ist.

Als Thermodesorptionsverfahren können deshalb übliche Thermodesorptionsverfahren zum Einsatz kommen, die durch direkte strahlungs- und/oder kontaktbasierende Wärmeübergänge motiviert werden. Auch können Plasmadesorptionsverfahren, bei dem heiße oder kalte atmosphärische Gas- Plasmen eine Partikeldesorption von der Glas-Oberfläche ermöglichen, verwendet werden. Auch können Laser-Desorptionsverfahren verwendet werden, die bei geeigneten Wellenlängen im Absorptionsbereich der Sprengstoffe eine selektive Desorption der interessierenden Stoffe ermöglichen.

Grundsätzlich wird entweder der Detektor zur Scanner-Einheit oder die Scanner-Einheit zum Detektor transportiert. Weiterhin kann durch eine Art Drehscheiben-Konstruktion eine elegante Trennung von Sampeln, Reinigen und Messung erfolgen.

Der Gefahrstoff kann mittels einer Saugvorrichtung von der transparenten Trägerplatte abgesaugt werden.

Weitere Aspekte der Erfindung ergeben sich aus der folgenden Figurenbeschreibung. Im Folgenden werden mit Verweis auf die Figuren Ausführungsbeispiele der Erfindung beschrieben.

### Kurze Beschreibung der Figuren

Fig. 1 zeigt einen integrierten Fingerabdruck- und Gefahrstoff-Sensor gemäß einem Ausführungsbeispiel der Erfindung.
Fig. 2 zeigt eine automatische Personenschleuse gemäß einem weiteren Ausführungsbeispiel der Erfindung.
Fig. 3 zeigt Teile eines integrierten Fingerabdruck- und Gefahrstoff-Sensors gemäß einem weiteren Ausführungsbeispiel der Erfindung.
Fig. 4 zeigt eine schematische Darstellung einer Personenschleuse gemäß einem weiteren Ausführungsbeispiel der Erfindung.

Die Darstellungen in den Figuren sind schematisch und nicht maßstäblich.

Werden in der folgenden Figurenbeschreibung in verschiedenen Figuren die gleichen Bezugszeichen verwendet, so bezeichnen diese gleiche oder ähnliche Elemente. Gleiche oder ähnliche Elemente können aber auch durch unterschiedliche Bezugszeichen bezeichnet sein.

### Detaillierte Beschreibung von Ausführungsbeispielen

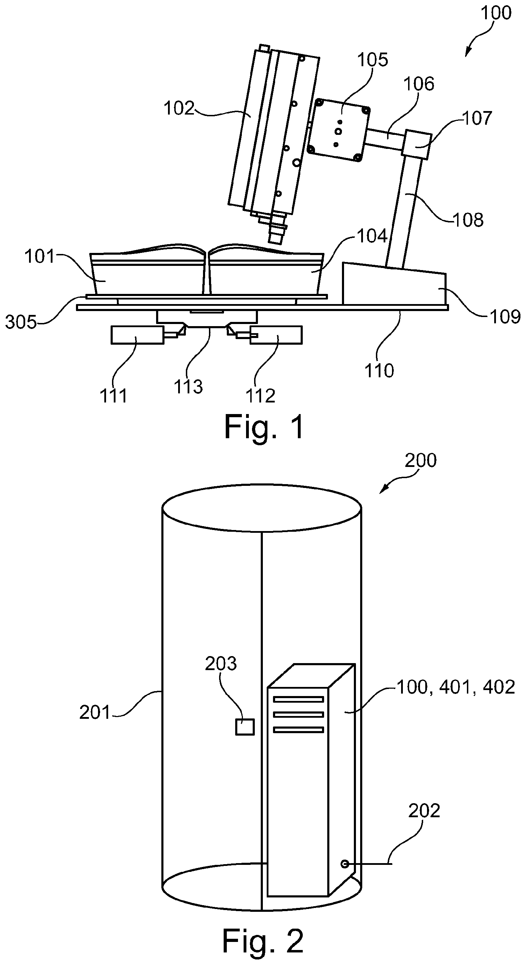

Fig. 1 zeigt einen integrierten Fingerabdruck- und Gefahrstoff-Sensor 100 zur Sicherheitskontrolle von Personen, beispielsweise zur Sicherheitskontrolle in einem Flughafen, einem Atomkraftwerk, einem Regierungsgebäude oder einer anderen Einrichtung mit erhöhten Sicherheitsanforderungen.

Der Sensor 100 weist eine, zwei oder mehrere Fingerabdruck- oder Handflächen-Scaneinrichtungen 101, 104 auf, die beispielsweise auf einem Drehteller 305 oder einer anderweitigen Transportvorrichtung angeordnet sind. Diese Scaneinrichtungen können ausgeführt sein, den Abdruck eines Fingers, einer Hand oder ggf. eines anderen Körperteils einer Person aufzunehmen und sind aus diesem Grund mit einer Datenverarbeitungsanlage verbunden (nicht dargestellt in Fig. 1). Die Datenverarbeitungsanlage kann entfernt von der Scaneinrichtung angeordnet sein. Nach Aufnahme des Finger- bzw. Handabdrucks wird der entsprechende Scanner 101, 104 in Richtung der Gefahrstoff-Sensoreinrichtung 102 bewegt, indem der Drehteller rotiert wird. In anderen Worten wird der Abdruck in der ersten Position des Scanners 101 aufgenommen, wonach der Scanner in die Position des anderen Scanners 104 rotiert bzw. anderweitig bewegt wird. Demnach wird der Fingerabdruck- oder Handflächenscanner nach Aufnahme der Fingerabdrücke in den hinteren Teil des Gerätes gedreht. Dort fährt der Gefahrstoff-Sensor 102 auf die mit den Fingerabdrücken belegte Sensorplatte. Diese wird aufgeheizt und die dadurch desorbierten Spuren dem Gefahrstoff-Sensor zur Analyse zugeleitet.

An diesem Ort kann dann die Gefahrstoff-Sensoreinrichtung 102 den Gefahrstoff detektieren.

Die Gefahrstoff-Sensoreinrichtung 102 ist beispielsweise über den Positionierer 105, einem ersten Tragarmabschnitt 106, einem Gelenk 107 und einem zweiten, vertikalen Armabschnitt 108 mit dem Standfuß 109 verbunden, der an einem Basiselement 100 befestigt ist, welches sowohl die Scaneinrichtungen 101, 104 mit dem Drehteller als auch die Gefahrstoff-Sensoreinrichtung 102 trägt.

Unter der Platte 110 befinden sich die Elemente 111, 112, 113, bei denen es sich um die Lagerung und den Antrieb des Drehtellers handelt.

Fig. 2 zeigt eine automatische Personenschleuse 200 gemäß einem weiteren Ausführungsbeispiel. Diese Schleuse 200 weist einen oder mehrere der oben und unten beschriebenen Fingerabdruck- und Gefahrstoff-Sensoren 100 auf. Darüber hinaus kann eine Dokumenten-Scaneinrichtung 401 vorgesehen sein, mit deren Hilfe Personendaten aus einem Dokument eingelesen und überprüft werden können.

Auch kann beispielsweise ein Gamma-Detektor 402 vorgesehen sein, um Gamma-Strahlung zu detektieren.

Darüber hinaus kann an der Außenseite der Personenschleuse 200 ein Bordkartenleser 203 angeordnet sein, um den Zugang autorisierter Personen in die Schleuse zu ermöglichen und den Zugang nicht autorisierter Personen zu verhindern.

Hierfür weist die Schleuse 200 beispielsweise eine Drehtür 201 auf, welche entsprechend geöffnet und verschlossen und verriegelt werden kann. Öffnen und Verschließen können vollautomatisch auf Basis der entsprechenden Messdaten erfolgen.

Die Einrichtungen 100, 401, 402 können über die Datenleitung 202 mit externen Recheneinheiten einer Datenverarbeitungsanlage verbunden sein, welche die Daten auswerten und die automatische Personenschleuse steuern.

Somit werden die Prozessschritte der Sicherheitskontrolle (Gefahrstoffdetektion, Detektion von verbotenen Gegenständen, Identifikation einer Person oder Verifikation eines Dokuments) nicht mehr getrennt an verschiedenen Orten durchgeführt und können miteinander korreliert werden, da sämtliche Messdaten einer zentralen Recheneinheit zugeführt werden können. Hierdurch können Mehrfachkontrollen und signifikante Erkenntnisverluste vermieden werden. Darüber hinaus kann die Effizienz der Sicherheitskontrolle erhöht werden.

Die Gefahrstoffkontrolle, die durch die Gefahrstoff-Sensoreinrichtung, ggf. in Kombination mit weiteren Sensoren erfolgt, geschieht beispielsweise auf Basis der IMS-Technologie (Ionen-Mobilitäts-Spektrometrie) oder Gaschromatographie.

Auch können teilautomatisierte Einrichtungen verwendet werden, welche für die Detektion Luftproben aus dem Innenraum der Personenschleuse entnehmen. Die Identifikation der Person kann beispielsweise auch auf Basis von Iriskontrolle alternativ oder zusätzlich zur Fingerabdruckkontrolle erfolgen. In diesem Fall muss die Person zwar keine Stoffprobe auf die Oberfläche eines Fingerabdrucksensors abgeben, allerdings kann vorgesehen sein, dass die Person eine andere Oberfläche berühren muss, damit eine Stoffprobe abgegeben wird.

Die automatische Personenschleuse 200 ist demnach mit verschiedenen Sensoren zur Identitätskontrolle und der Verifikation von Ausweis-Dokumenten sowie zur Detektion von Gefahrstoffen ausgestattet. Dies kann durch eine Kombination der als sehr zuverlässig geltenden Personenidentifikation durch Fingerabdruck-Lesegeräte mit ebenfalls bewährten Gefahrstoffsensoren, die Spuren derartiger Stoffe in den Fingerabdrücken nachweisen können, erfolgen.

Somit können die aus den Messungen gewonnenen Erkenntnisse miteinander korreliert werden und eindeutig der kontrollierten Person zugeordnet werden.

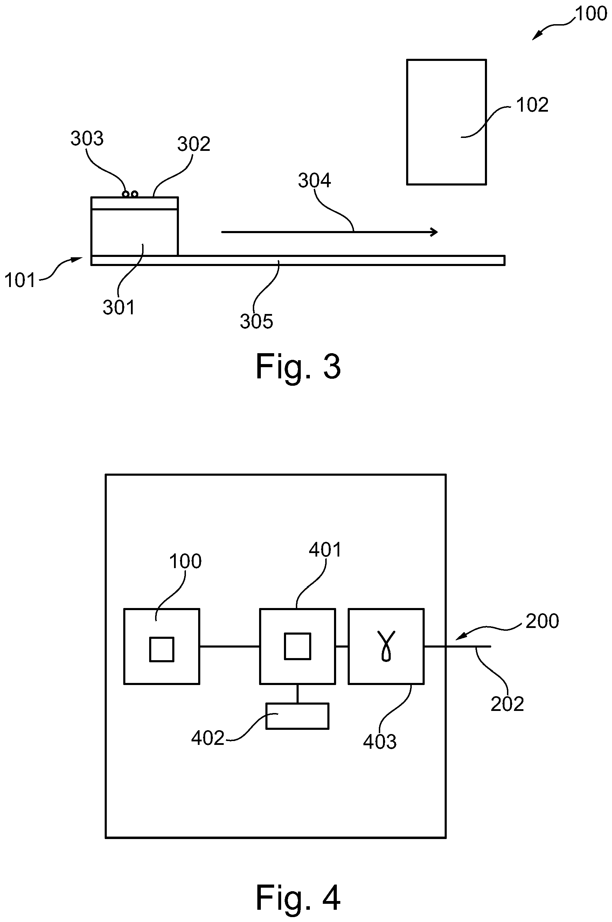

Fig. 3 zeigt Bauteile eines integrierten Fingerabdruck- und Gefahrstoff-Sensors 100 gemäß einem Ausführungsbeispiel der Erfindung. Die Fingerabdruck- oder Handflächen-Scaneinrichtung 101 ist auf einer Transporteinrichtung 305 angeordnet, bei der es sich beispielsweise um einen Drehteller oder eine lineare Transporteinrichtung handeln kann. Die Scaneinrichtung 101 weist eine optische Detektionsvorrichtung 301 auf, auf der sich eine transparente Platte 302, beispielsweise eine Glasplatte, befindet. Wird der Finger auf die Glasplatte 302 aufgelegt, kann der Gefahrstoff vom Finger abgegeben werden und beispielsweise in Form der Partikel 303 auf der Oberfläche 302 adsorbiert werden.

Daraufhin wird die Scaneinrichtung 101 in Richtung des horizontalen Pfeils 304 zur Gefahrstoff-Sensoreinrichtung 102 transportiert, welche nahe an die Stoffprobe 303 herangeführt wird (oder umgekehrt), um den Gefahrstoff zu detektieren.

Fig. 4 zeigt eine Anordnung aus einem integriertem Fingerabdruck- und Gefahrstoff-Sensor 100, einem Dokumenten-Scanner 401, einem Gamma-Detektor 403 und einem Bordkartenleser 402. Diese verschiedenen Sensoren, Scaneinrichtungen und Detektoren sind über einen Datenbus 202 miteinander verbunden und die gesammelten Daten werden über den Bus 202 an eine zentrale Recheneinheit übermittelt, die die Daten miteinander korrelieren, analysieren, auswerten und ggf. weitergeben kann. Weiterhin kann die zentrale Recheneinheit daraufhin notwendige Schritte einleiten, je nach dem Analyseergebnis der Daten. Beispielsweise kann ein Alarm ausgelöst werden oder es kann vorgesehen werden, dass weitere Datenaufzeichnungen über die Sensoren, die in Fig. 4 gezeigt sind, erfolgen, um genauere Informationen über die Person und/oder den möglichen Gefahrstoff zu erhalten.

Durch die automatische Personenschleuse 200 kann der Zeitaufwand, der bei Sicherheitskontrollen oft erforderlich ist, verringert werden. Darüber hinaus können sämtliche Messungen an ein und demselben Ort durchgeführt werden, was den Gesamtprozess zusätzlich beschleunigt und vereinfacht.

Beispielsweise erfolgt demnach eine Kombination der als sehr zuverlässig geltenden Personenidentifikation durch ein Fingerabdruck-Lesegerät und Gefahrstoff-Sensoren, die Spuren von Gefahrstoffen in den Fingerabdrücken nachweisen können. Somit erfolgt also eine Kombination von Fingerabdruck-Identifikation und Spurendetektion.

Die im Wesentlichen für die Gefahrstoffdetektion relevanten Stoffe haften besonders gut an Hand- und Fingeroberflächen von Personen, die zuvor im näheren Kontakt mit diesen standen. Nach Berühren der Personen-Identifikationshandscannerflächen (meist Glas) wird ein gewisser Anteil der Partikelfracht auf diesen Glas-Flächen hinterlassen und kann zur weiteren Identifikation verwendet werden.

Auf diese Weise kann sehr effizient, vereinfacht gegenüber bekannten Verfahren und auch sehr genau eine relevante Spurenanalyse durchgeführt werden.

Folgende Gefahren- und Stoffgruppen können für das geschilderte Verfahren und insbesondere für die automatisierte Personenschleuse zum Einsatz kommen:
- Für die Detektion von relevanten chemischen Stoffgruppen für die Gefahrstoffdetektion schwerflüchtiger Verbindungen (wie schwerflüchtige Explosivstoffe, d.h. Plastiksprengstoffe oder schwerflüchtige Toxische Industrie-Chemikalien (TICs) oder auch Drogen) können insbesondere Thermodesorptionsverfahren eingesetzt werden, welche den partikelförmigen Gefahrstoff auf der Glasoberfläche des Finger- oder Handabdruckscanners in die Gasphase überführen können. Aus dieser gasförmigen Phase kann dann relativ einfach und effizient eine weitere Analyse der Stoffgruppen mit bekannten chemischen bzw. elektrochemischen Analysegeräten durchgeführt werden.
   Zur Erhöhung der Gesamtselektivität der Anordnung kann eine substanzspezifische Desorption der relevanten Partikel durchgeführt werden. Hierfür kommen selektive Photonenanregungen bzw. elektromagnetische Anregungsspektren und Verfahren in Frage.
- Zur Erweiterung der Detektion von gasförmigen Gefahrstoffen (wie leichtflüchtige Sprengstoffe, z.B. Flüssigsprengstoffe oder mittel- und leichtflüchtige Toxische Industrie Chemikalien) kann die Gasphase zeitlich vor dem Einsatz des Thermodesorptionsverfahrens untersucht werden. Zusätzlich kann durch zusätzliche Absaugung der Umgebungsluft um die Hand oder die Person die Luft auf diese gasförmigen Gefahrstoffe untersucht werden.
- Partikelförmige Radioisotope bzw. Partikel, wie sie nach atomaren Katastrophen oder terroristischen Aktionen (sog. Dirty Bomb) auftreten können, verbinden sich auch hier oft mit der Hautoberfläche von involvierten Personen. Somit kann eine Identifikation der speziellen Stoffgruppen von der Glasoberfläche erfolgen. Allerdings in diesem Fall beispielsweise ohne vorherige Desorption, sondern durch eine Strahlungsnahfelddetektion der relevanten Alpha- und Beta-Strahlung von den Glasoberflächen. Beispielsweise steht in der Detektionsreihenfolge die Strahlungsdetektion an erster Stelle, gefolgt von beispielsweise dem Thermodesorptionsverfahren.
- Bioaktive Substanzen, wie sie in entsprechenden B-Kampfstoffszenarien oder bei Epidemien auftreten können, haften ebenfalls an der Hautoberfläche und können auch hier mittels entsprechender Sensorik für die weitere Analyse in Betracht gezogen werden. Hierzu eignen sich vorteilhaft Verfahren, wie die Raman-Analyse oder auch Antikörper-basierende Verfahren. Je nach Anwendung können entsprechende Analysegeräte vorgesehen sein.

Hierdurch kann bereits im Vorfeld, d. h. vor Betreten des Sicherheitsbereichs, eine Zugangsbegrenzung ermöglicht werden. Entsprechende identifizierte Personen können demzufolge dann einer weiteren oder genaueren Kontrolle unterzogen werden. Auf diese Weise können relevante Gefährdungspotenziale minimiert werden.

An dieser Stelle sei darauf hingewiesen, dass die aufgenommenen Sensordaten mit zentralen Datenbanken abgeglichen werden können. Hierfür werden die Daten über die Kommunikationsleitung 202 (kabelgebunden oder kabellos) an den Zentralrechner übertragen, der mit den Datenbanken in Verbindung steht (siehe Fig. 2).

## Patentansprüche

1. Integrierter Fingerabdruck- und Gefahrstoff-Sensor (100) zur Sicherheitskontrolle von Personen, der Sensor aufweisend:
eine Fingerabdruck- oder Handflächen-Scaneinrichtung (101) zur Aufnahme eines Abdrucks eines Fingers oder einer Hand einer Person;
eine Gefahrstoff-Sensoreinrichtung (102) zur Detektion eines Gefahrstoffs (303), der von der Person während der Aufnahme des Abdrucks an die Fingerabdruck- oder Handflächen-Scaneinrichtung (101) abgegeben wurde; und
eine Transportvorrichtung (305) zum Transport
- der Fingerabdruck- oder Handflächen-Scaneinrichtung (101) in einen Wirkbereich der Gefahrstoff-Sensoreinrichtung (102), nachdem die Aufnahme des Abdrucks erfolgt ist, oder
- der Gefahrstoff-Sensoreinrichtung (102) in Richtung der Fingerabdruck- oder Handflächen-Scaneinrichtung (101), so dass die Fingerabdruck- oder Handflächen-Scaneinrichtung (101) in einen Wirkbereich der Gefahrstoff-Sensoreinrichtung (102) gelangt, nachdem die Aufnahme des Abdrucks erfolgt ist.

2. Integrierter Sensor nach Anspruch 1,
wobei die Fingerabdruck- oder Handflächen-Scaneinrichtung (101) eine Oberfläche (302) zum Anlegen des Fingers oder der Hand während der Aufnahme des Abdrucks aufweist, auf welcher sich der Gefahrstoff bei Anlegen des Fingers oder der Hand ablagert; und
wobei die Gefahrstoff-Sensoreinrichtung (102) zur anschließenden, automatischen Identifikation des Gefahrstoffs ausgeführt ist.

3. Integrierter Sensor nach Anspruch 1 oder 2,
wobei die Transportvorrichtung (305) einen Drehteller aufweist, auf welchem die Fingerabdruck- oder Handflächen-Scaneinrichtung (101) angeordnet ist und mit welchem sie in den Wirkbereich der Gefahrstoff-Sensoreinrichtung (102) hineingedreht wird.

4. Integrierter Sensor nach einem der vorhergehenden Ansprüche,
wobei die Gefahrstoff-Sensoreinrichtung (102) zur Durchführung eines Thermodesorptionsverfahrens zur Überführung des Gefahrstoffs auf der Fingerabdruck- oder Handflächen-Scaneinrichtung (101) in eine Gasphase zur weiteren Analyse des Gefahrstoffs ausgeführt ist.

5. Integrierter Sensor nach einem der vorhergehenden Ansprüche,
wobei die Gefahrstoff-Sensoreinrichtung (102) zur Durchführung einer chemischen oder einer elektrochemischen Analyse des Gefahrstoffs ausgeführt ist.

6. Integrierter Sensor nach einem der vorhergehenden Ansprüche,
wobei die Gefahrstoff-Sensoreinrichtung (102) zur Detektion von radioaktiver Strahlung, die von dem Gefahrstoff emittiert wird, ausgeführt ist.

7. Integrierter Sensor nach Anspruch 6,
wobei die Gefahrstoff-Sensoreinrichtung (102) zur Detektion von radioaktiver α- und β-Strahlung ausgeführt ist, so dass auch eine Detektion von beispielsweise Plutonium als Gefahrstoff ermöglicht wird.

8. Integrierter Sensor nach einem der vorhergehenden Ansprüche,
wobei die Gefahrstoff-Sensoreinrichtung (102) zur Detektion von bioaktiven Substanzen ausgeführt ist.

9. Automatische Personenschleuse (200) zur Sicherheitskontrolle von Personen, aufweisend einen integrierten Fingerabdruck- und Gefahrstoff-Sensor (100) nach einem der Ansprüche 1 bis 8.

10. Automatische Personenschleuse nach Anspruch 9, weiterhin aufweisend:
eine Dokumenten-Scaneinrichtung (401) zum Einlesen und Überprüfen von Personendaten aus einem Dokument.

11. Automatische Personenschleuse nach einem der Ansprüche 9 oder 10,
ausgeführt zur Verhinderung des Durchgangs der Person durch die Schleuse, wenn der Fingerabdruck- und Gefahrstoff-Sensor (100) einen Gefahrstoff detektiert.

12. Automatische Personenschleuse nach Anspruch 11,
ausgeführt zur Verhinderung des Durchgangs der Person durch die Schleuse nur dann, wenn die Konzentration des detektierten Gefahrstoffs eine Mindestkonzentration übersteigt.

13. Verwendung eines integrierten Fingerabdruck- und Gefahrstoff-Sensors (100) nach einem der Ansprüche 1 bis 8 zur Sicherheitskontrolle in einem Flughafen.

## Claims

1. Integrated fingerprint and hazardous substance sensor (100) for security checks on individuals, the sensor comprising:
a fingerprint or palm-scanning apparatus (101) for capturing a print of a finger or a hand of an individual;
a hazardous substance sensor apparatus (102) for detecting a hazardous substance (303) that was left by the individual on the fingerprint or palm-scanning apparatus (101) whilst the print was being captured; and
a transport apparatus (305) for transporting
- the fingerprint or palm-scanning apparatus (101) into an effective region of the hazardous substance sensor apparatus (102) after the print has been captured, or for transporting
- the hazardous substance sensor apparatus (102) towards the fingerprint or palm-scanning apparatus (101), so that the fingerprint or palm-scanning apparatus (101) enters an effective region of the hazardous substance sensor apparatus (102) after the print has been captured.

2. Integrated sensor according to claim 1,
wherein the fingerprint or palm-scanning apparatus (101) comprises a surface (302) for placing the finger or the hand whilst the print is being captured and on which the hazardous substance settles when the finger or hand is placed thereon; and
wherein the hazardous substance sensor apparatus (102) is designed to subsequently automatically identify the hazardous substance.

3. Integrated sensor according to either claim 1 or claim 2,
wherein the transport apparatus (305) comprises a rotary table, on which the fingerprint or palm-scanning apparatus (101) is arranged and by means of which said scanning apparatus is rotated into the effective region of the hazardous substance sensor apparatus (102).

4. Integrated sensor according to any of the preceding claims,
wherein the hazardous substance sensor apparatus (102) is designed to carry out a thermal desorption method for converting the hazardous substance on the fingerprint or palm-scanning apparatus (101) into a gas phase for further analysis of the hazardous substance.

5. Integrated sensor according to any of the preceding claims,
wherein the hazardous substance sensor apparatus (102) is designed to perform a chemical or an electrochemical analysis of the hazardous substance.

6. Integrated sensor according to any of the preceding claims,
wherein the hazardous substance sensor apparatus (102) is designed to detect radioactive radiation that is emitted by the hazardous substance.

7. Integrated sensor according to claim 6,
wherein the hazardous substance sensor apparatus (102) is designed to detect radioactive α and β radiation, so as to also permit detection of plutonium, for example, as the hazardous substance.

8. Integrated sensor according to any of the preceding claims,
wherein the hazardous substance sensor apparatus (102) is designed to detect bioactive substances.

9. Automatic turnstile (200) for security checks on individuals, comprising an integrated fingerprint and hazardous substance sensor (100) according to any of claims 1 to 8.

10. Automatic turnstile according to claim 9, further comprising:
a document-scanning apparatus (401) for reading and verifying personal data from a document.

11. Automatic turnstile according to either claim 9 or claim 10,
designed to prevent the individual from passing through the turnstile when the fingerprint and hazardous substance sensor (100) detects a hazardous substance.

12. Automatic turnstile according to claim 11,
designed to prevent the individual from passing through the turnstile only when the concentration of the detected hazardous substance exceeds a minimum concentration.

13. Use of an integrated fingerprint and hazardous substance sensor (100) according to any of claims 1 to 8 for security checks in an airport.

## Revendications

1. Détecteur intégré d'empreintes digitales et de matières dangereuses (100) pour le contrôle de sécurité de personnes, le détecteur comprenant :
un dispositif scanner d'empreintes digitales ou de paumes de la main (101) pour la capture de l'empreinte d'un doigt ou d'une main d'une personne ;
un dispositif détecteur de matières dangereuses (102) pour la détection d'une matière dangereuse (303) qui a été fournie par la personne sur le dispositif scanner d'empreintes digitales ou de paumes de la main (101) pendant la capture de l'empreinte ; et
un dispositif de transport (305) pour le transport
- du dispositif scanner d'empreintes digitales ou de paumes de la main (101) dans une zone active du dispositif détecteur de matières dangereuses (102) après que la capture de l'empreinte a été effectuée, ou
- du dispositif détecteur de matières dangereuses (102) en direction du dispositif scanner d'empreintes digitales ou de paumes de la main (101) de telle sorte que le dispositif scanner d'empreintes digitales ou de paumes de la main (101) arrive dans une zone active du dispositif détecteur de matières dangereuses (102), après que la capture de l'empreinte a été effectuée.

2. Détecteur intégré selon la revendication 1,
dans lequel le dispositif scanner d'empreintes digitales ou de paumes de la main (101) présente une surface (302) servant à l'application du doigt ou de la main pendant la capture de l'empreinte, sur laquelle la matière dangereuse se dépose lors de l'application du doigt ou de la main ; et
dans lequel le dispositif détecteur de matières dangereuses (102) est conçu pour l'identification automatique subséquente de la matière dangereuse.

3. Détecteur intégré selon la revendication 1 ou 2,
dans lequel le dispositif de transport (305) présente un plateau tournant, sur lequel le dispositif scanner d'empreintes digitales ou de paumes de la main (101) est disposé et au moyen duquel celui-ci est amené par rotation dans la zone active du dispositif détecteur de matières dangereuses (102).

4. Détecteur intégré selon l'une quelconque des revendications précédentes,
dans lequel le dispositif détecteur de matières dangereuses (102) est conçu pour l'exécution d'un procédé de thermodésorption pour le passage de la matière dangereuse sur le dispositif scanner d'empreintes digitales ou de paumes de la main (101) dans une phase gazeuse en vue d'une analyse complémentaire de la matière dangereuse.

5. Détecteur intégré selon l'une quelconque des revendications précédentes,
dans lequel le dispositif détecteur de matières dangereuses (102) est conçu pour l'exécution d'une analyse chimique ou d'une analyse électrochimique de la matière dangereuse.

6. Détecteur intégré selon l'une quelconque des revendications précédentes,
dans lequel le dispositif détecteur de matières dangereuses (102) est conçu pour la détection d'un rayonnement radioactif émis par la matière dangereuse.

7. Détecteur intégré selon la revendication 6,
dans lequel le dispositif détecteur de matières dangereuses (102) est conçu pour la détection d'un rayonnement radioactif α et β, de telle sorte qu'il est possible de détecter également par exemple du plutonium comme matière dangereuse.

8. Détecteur intégré selon l'une quelconque des revendications précédentes,
dans lequel le dispositif détecteur de matières dangereuses (102) est conçu pour la détection de substances bioactives.

9. Tourniquet automatique (200) pour le contrôle de sécurité de personnes, présentant un détecteur intégré d'empreintes digitales et de matières dangereuses (100) selon l'une quelconque des revendications 1 à 8.

10. Tourniquet automatique selon la revendication 9, présentant en outre :
un dispositif scanner de documents (401) pour la lecture et la vérification de données personnelles à partir d'un document.

11. Tourniquet automatique selon l'une quelconque des revendications 9 ou 10,
conçu pour empêcher le passage de la personne par le tourniquet si le détecteur intégré d'empreintes digitales et de matières dangereuses (100) détecte une matière dangereuse.

12. Tourniquet automatique selon la revendication 11,
conçu pour empêcher le passage de la personne par le tourniquet seulement lorsque la concentration de la matière dangereuse détectée dépasse une concentration minimale.

13. Utilisation d'un détecteur intégré d'empreintes digitales et de matières dangereuses (100) selon l'une quelconque des revendications 1 à 8 pour le contrôle de sécurité dans un aéroport.
